# EUROPÄISCHE PATENTSCHRIFT

(11) **EP 2 269 522 B1**
(45) Veröffentlichungstag und Bekanntmachung des Hinweises auf die Patenterteilung: **02.08.2017**
(21) Anmeldenummer: 10006016.9
(22) Anmeldetag: 10.06.2010
(51) Int. Cl.: A61B 17/29

(54) **Medizinisches Instrument**
Medical instrument
Instrument médical

(30) Priorität: 30.06.2009 DE 102009031262
(43) Veröffentlichungstag der Anmeldung: 05.01.2011
(73) Patentinhaber: Karl Storz GmbH & Co. KG, 78532 Tuttlingen (DE)
(72) Erfinder: Reinauer, Josef, 72514 Inzigkofen (DE)

(56) Entgegenhaltungen:
- EP-A1- 0 571 057
- WO-A1-2008/118945
- DE-A1- 3 739 254
- DE-A1- 3 741 879
- DE-A1- 19 731 453

## Beschreibung

Die Erfindung betrifft ein medizinisches Instrument mit einen Schaft, an dessen distalem Ende ein mindestens ein bewegbares Maulteil aufweisendes Werkzeug angeordnet ist und an dessen proximalem Ende eine mit mindestens einem bewegbaren Griffteil versehene Handhabe angeordnet ist, wobei das bewegbare Maulteil sowie das bewegbare Griffteil über ein Zug-/Druckelement miteinander in Wirkverbindung stehen und wobei zwischen dem bewegbaren Griffteil und dem bewegbaren Maulteil mindestens eine Kraftbegrenzungsvorrichtung zum Begrenzen der bei der Kraftübertragung von dem bewegbaren Griffteil auf die mit dem bewegbaren Griffteil in Wirkverbindung stehenden Komponenten übertragenen Kraft angeordnet ist, die bei Überschreitung einer Grenzlast die miteinander gekoppelten Komponenten reversibel trennt.

Bei derartigen medizinischen Instrumenten kann es sich beispielsweise um Fass-, Halte- und Präparierzangen, Scheren oder dergleichen Instrumente handeln, bei denen durch Handkraft über ein verstellbares Griffteil der Handhabe ein verstellbares Werkzeug, beispielsweise ein verschwenkbares Maulteil, bewegt wird.

Diese bekannten medizinischen Instrumente, die beispielsweise in der endoskopischen Chirurgie Verwendung finden, weisen einen Schaft auf, an dessen distalem Ende ein mindestens ein bewegbares Maulteil aufweisendes Werkzeug angeordnet ist und an dessen proximalem Ende eine mit mindestens einem bewegbaren Griffteil versehene Handhabe angeordnet ist. Zum Öffnen und Schließen des bewegbaren Maulteils sind das bewegbare Maulteil und das bewegbare Griffteil über ein Zug-/Druckelement miteinander verbunden. Die Griffteile der Handhaben sind in der Regel geometrisch so ausgebildet, dass die über die Hand des Benutzers auf das Griffteil ausgeübte Kraft bei der Übertragung auf das mit dem Maulteil gekoppelte Zug-/Druckelement deutlich verstärkt wird, beispielsweise im Verhältnis 10:1, um auch ohne große Kraftanstrengung eine ausreichend hohe Schließkraft am Maulteil zu erzielen.

Um zu verhindern, dass über die Handhabe so große Kräfte auf die Maulteile und/oder das Zug-/Druckelement ausgeübt werden, die zu einer Beschädigung oder gar Zerstörung einzelner Bauteile führen können, ist es aus der Praxis bekannt, medizinische Instrumente mit einer Kraftbegrenzungsvorrichtung zu versehen, die bei Überschreitung einer Grenzlast die miteinander gekoppelten Komponenten reversibel trennt.

Hierzu ist es beispielsweise bekannt, den Kraftübertragungsmechanismus mit einer Sollbruchstelle auszustatten. Diese Art der Kraftbegrenzung gewährleistet zwar einen sicheren Schutz des Instruments, beinhaltet aber den Nachteil, dass das Instrument nicht sofort wieder einsatzfähig ist, da es zur Reparatur der Sollbruchstelle demontiert werden muss.

Ein gattungsgemäßes medizinisches Instrument mit einer reversibel arbeitenden Kraftbegrenzungsvorrichtung ist beispielsweise aus der DE 197 31 453 C2 bekannt. Bei diesem bekannten medizinischen Instrument ist die Kraftbegrenzung als elastisch verformbarer Teil des Zug-/Druckelements ausgebildet, der sich bei Überschreiten der Grenzlast reversibel verformt. Weiterhin ist eine Kraftbegrenzungsvorrichtung (s. Abb. 5) für ein endoscopisches Instrument aus WO 2008/118945 bekannt. Davon ausgehend liegt der Erfindung die **Aufgabe** zugrunde, ein medizinisches Instrument mit einer Kraftbegrenzungsvorrichtung zu schaffen, die bei Erreichen der Grenzlast ein deutlich erkennbares Herabsetzen der ausgeübten Kraft gewährleistet. Die Aufgabe wird durch die Merkmale des Anspruchs 1 gelöst. Die erfindungsgemäße Ausbildung der Kraftbegrenzungsvorrichtung mit den zwei relativ zueinander verschiebbaren, vorteilhafterweise hülsenförmig ausgebildeten Bauteilen hat den Vorteil, dass das federbelastete axial verschiebbare Bauteil bei Überschreiten der Grenzlast durch das mindestens eine Kraftübertragungselement schlagartig weggedrückt wird, so dass die über das verstellbare Griffteil der Handhabe ausgeübte Kraft schlagartig quasi auf Null absinkt.

Gemäß der Erfindung ist das mindestens eine Kraftübertragungselement, über das die Zugkraft des verstellbaren Griffteils der Handhabe auf die beiden Bauteile übertragbar ist, als Kugel ausgebildet. Diese Art des Kraftübertragungselements ist besonders einfach herzustellen und zu montieren. Weiterhin wird mit der Erfindung vorgeschlagen, dass die Anlageflächen, an denen das mindestens eine Kraftübertragungselement an den beiden Bauteilen anliegt, als aufeinander zu geneigte Schrägflächen so ausgebildet sind, dass die Grenzlast zum Auslösen der Kraftbegrenzungsvorrichtung eine Winkelfunktion der Kraft des Kraftübertragungselements auf die schräge Anlagefläche des verschiebbaren Bauteils ist, wobei die Kraft des Kraftübertragungselements auf die schräge Anlagefläche des verschiebbaren Bauteils eine Winkelfunktion der über das verstellbare Griffteil der Handhabe auf die schräge Anlagefläche des starren Bauteils ausgeübten Zugkraft ist.

Zur Anordnung der Kraftbegrenzungsvorrichtung wird gemäß einer bevorzugten Ausführungsform der Erfindung vorgeschlagen, dass diese im bewegbaren Griffteil der Handhabe im Bereich des Kraftübertragungsmechanismus auf das Zug-/Druckelement angeordnet ist. Die Anordnung der Kraftbegrenzungsvorrichtung in der Handhabe ist insbesondere bei endoskopischen Instrumenten vorteilhaft, da bei diesen Instrumenten aufgrund der schlanken Ausgestaltung der Schäfte weniger im Bereich der Schäfte, als vielmehr insbesondere im Bereich der Handhabe ausreichend Platz zur Unterbringung einer derartigen Vorrichtung vorhanden ist.

Die Übertragung der Zugkraft des verstellbaren Griffteils der Handhabe auf das mindestens eine Kraftübertragungselement erfolgt bei der in der Handhabe angeordneten Kraftübertragungsvorrichtung über einen am verstellbaren Griffteil der Handhabe gelagerten Zuganker.

Zur Anpassung des medizinischen Instruments an unterschiedliche Einsatzzwecke sowie zur Anpassung an unterschiedliche Werkzeugmaulteile ist die das Auslösen der Kraftbegrenzungsvorrichtung bewirkende Grenzlast über das auf das verschiebbare Bauteil einwirkende Federelement einstellbar.

Weiterhin wird mit der Erfindung vorgeschlagen, dass das Auslösen der Kraftbegrenzungsvorrichtung taktil und/oder akustisch und/oder optisch wahrnehmbar ist, um dem Benutzer zweifelsfrei anzuzeigen, dass die zulässige Grenzlast überschritten und die Kraftbegrenzungsvorrichtung ausgelöst wurde.

Schließlich wird mit einer alternativen Ausführungsform der Erfindung vorgeschlagen, dass die Kraftbegrenzungsvorrichtung im Bereich des Zug-/Druckelements angeordnet und beispielsweise als Teil des Zug-/Druckelements, ausgebildet ist.

Weitere Merkmale und Vorteile der Erfindung ergeben sich anhand der zugehörigen Zeichnungen, in denen zwei Ausführungsbeispiele eines erfindungsgemäßen medizinischen Instruments nur beispielhaft dargestellt sind, ohne die Erfindung auf diese Ausführungsbeispiele zu beschränken. In den Zeichnungen zeigt:
- Fig. 1: eine schematische Seitenansicht eines erfindungsgemäßen medizinischen Instruments;
- Fig. 2: eine vergrößerte und teilweise geschnittene schematische Darstellung der Handhabe eines medizinischen Instruments gemäß Fig. 1, den nicht ausgelösten Zustand der Kraftbegrenzungsvorrichtung darstellend;
- Fig. 3: eine Ansicht der Handhabe gemäß Fig. 2, jedoch den ausgelösten Zustand der Kraftbegrenzungsvorrichtung darstellend;
- Fig. 4: eine schematische Darstellung der Kraftverteilung innerhalb der Kraftbegrenzungsvorrichtung und
- Fig. 5: eine schematische Darstellung eines Kraftübertragungselements (nicht gemäß der Erfindung).

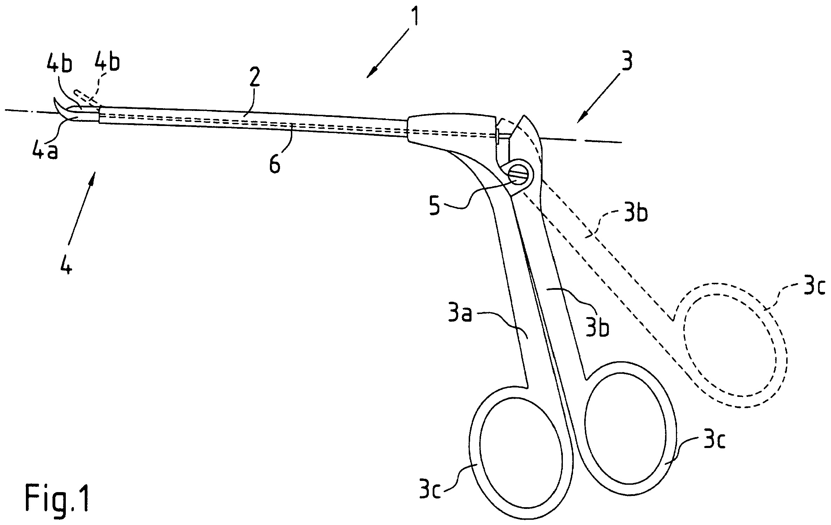

Die Abbildung Fig. 1 zeigt schematisch ein medizinisches Instrument, bei dem es sich beispielsweise um eine Fass-, Halte- oder Präparierzange, eine Schere oder dergleichen Instrument handeln kann, bei dem durch Handkraft ein Werkzeug, beispielsweise ein verschwenkbares Maulteil, bewegt wird.

Das nur schematisch dargestellte medizinische Instrument 1 besteht im Wesentlichen aus einem hohlen Schaft 2, an dessen proximalem Ende eine Handhabe 3 angeordnet ist, die aus einem starren Griffteil 3a und einem gegenüber dem starren Griffteil 3a verschwenkbaren Griffteil 3b besteht. Am distalen Ende des Schaftes 2 ist ein Werkzeug 4 angeordnet, das aus einem starren Maulteil 4a und einem gegenüber dem starren Maulteil 4a verschwenkbaren Maulteil 4b besteht. Welches der beiden Griffteile 3a und 3b der Handhabe 3 verschwenkbar oder anderweitig verstellbar ausgebildet ist, ist für die Funktionsweise des medizinischen Instruments 1 nicht relevant.

Wie weiterhin aus Fig. 1 ersichtlich, stehen das verschwenkbare Maulteil 4b des Werkzeugs 4 und der um eine Schwenkachse 5 verschwenkbare Griffteil 3b der Handhabe 3 über ein in dem hohlen Schaft 2 axial verschiebbar gelagertes Zug-/Druckelement 6 derart in Wirkverbindung miteinander, dass das Verstellen des Griffteils 3b der Handhabe 3 das verschwenkbare Maulteil 4b des Werkzeugs 4 von der geschlossenen Stellung (durchgezogene Darstellung gemäß Fig. 1) in die geöffnete Stellung (gestrichelte Darstellung gemäß Fig. 1) bzw. umgekehrt überführt. Die jeweils zugehörige Stellung des verschwenkbaren Griffteils 3b der Handhabe 3 ist in der Abbildung Fig. 1 ebenfalls durchgezogen (für die geschlossene Stellung) und gestrichelt (für die geöffnete Stellung) dargestellt.

Wie aus einer Zusammenschau der Abbildungen Fig. 1 bis 3 ersichtlich, ist das verschwenkbare Griffteil 3b über einen Kipphebel 7 mit dem axial verschiebbaren Zug-/Druckelement 6 verbunden und der Abstand von der Schwenkachse 5 zu der Stelle, an der der Kipphebel 7 mit dem axial verschiebbaren Zug-/Druckelement 6 verbunden ist, wesentlich kürzer als der Abstand von der Schwenkachse 5 zu der am äußeren Ende des verschwenkbaren Griffteils 3b angeordneten Fingeröse 3c. Das Übersetzungsverhältnis beträgt etwa 10:1, so dass die über die Hand des Benutzer ausgeübte Schließkraft auf das Zehnfache verstärkt wird.

Um zu verhindern, dass über die Handhabe 3 so große Kräfte auf die Maulteile 4a und 4b und/oder das Zug-/Druckelement 6 ausgeübt werden, die zu einer Beschädigung oder gar Zerstörung einzelner Bauteile führen können, weist das medizinische Instrument 1 eine Kraftbegrenzungsvorrichtung 8 auf, die bei Überschreitung einer Grenzlast die miteinander gekoppelten Komponenten reversibel trennt.

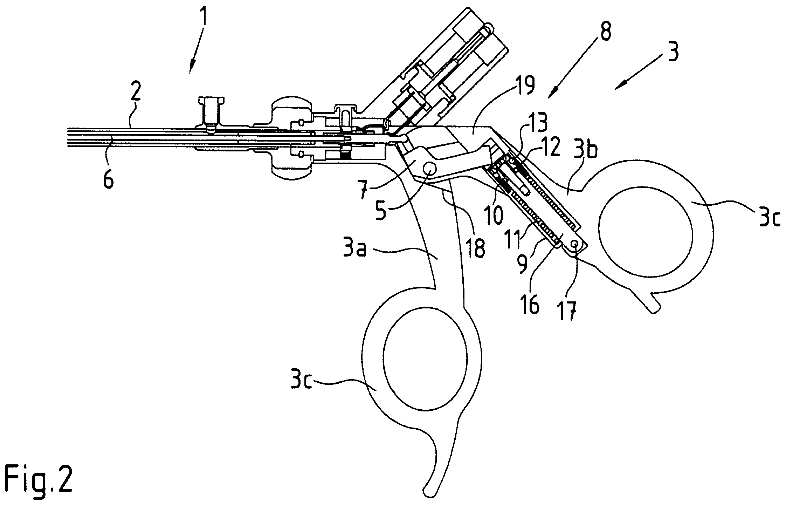

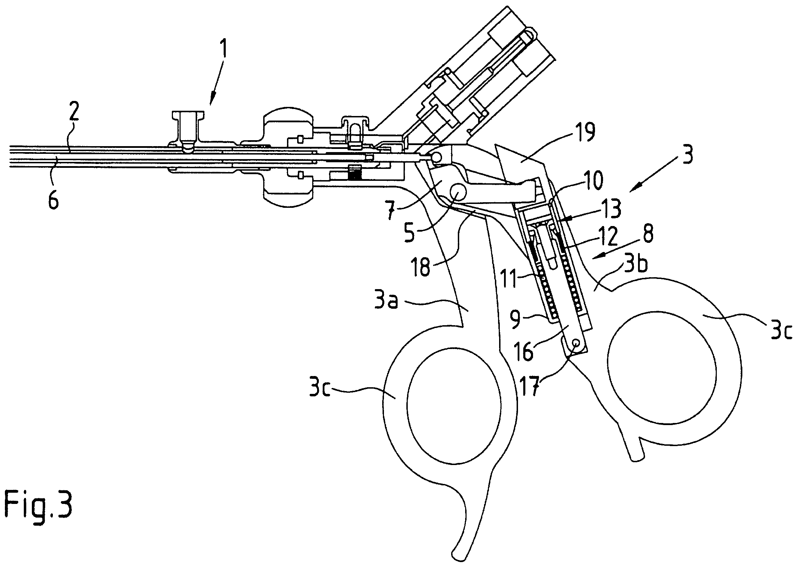

Bei der dargestellten Ausführungsform des medizinischen Instruments 1 ist die Kraftbegrenzungsvorrichtung 8, wie aus Fig. 2 und 3 ersichtlich, im bewegbaren Griffteil 3b der Handhabe 3 im Bereich des Kraftübertragungsmechanismus auf das Zug-/Druckelement 6 angeordnet.

Alternativ zu der dargestellten Anordnung der Kraftbegrenzungsvorrichtung 8 in der Handhabe 3 ist es auch möglich, die Kraftbegrenzungsvorrichtung 8 im Bereich des Zug-/Druckelements 6 anzuordnen und beispielsweise als Teil des Zug-/Druckelements 6 auszubilden.

Der Aufbau und die Arbeitsweise der Kraftbegrenzungsvorrichtung 8 ist den Abbildungen Fig. 2 bis 5 zu entnehmen.

Die Kraftbegrenzungsvorrichtung 8 besteht im Wesentlichen aus einem Gehäuse 9, in dem relativ zueinander verschiebbar ein starres Bauteil 10 und ein gegen die Kraft eines Federelements 11 axial verschiebbar im Gehäuse 9 gelagertes Bauteil 12 gelagert sind und wobei beide Bauteile 10 und 12 über ein auf beide Bauteile 10 und 12 einwirkendes Kraftübertragungselement 13 miteinander gekoppelt sind, das die Zugkraft F_{Z} des verstellbaren Griffteils 3b der Handhabe 3 auf die beiden Bauteile 10, 12 überträgt.

Wie aus Fig. 2 und 3 ersichtlich, sind die Anlageflächen 10a und 12a der beiden Bauteile 10 und 12, an denen das Kraftübertragungselement 13 an den beiden Bauteilen 10, 12 anliegt, als aufeinander zu geneigte Schrägflächen ausgebildet.

Das mindestens eine an den Anlageflächen 10a und 12a anliegende Kraftübertragungselement 13 ist bei der in den Abbildungen Fig. 2 bis 4 dargestellten ersten Ausführungsform als Kugel 14 ausgebildet.

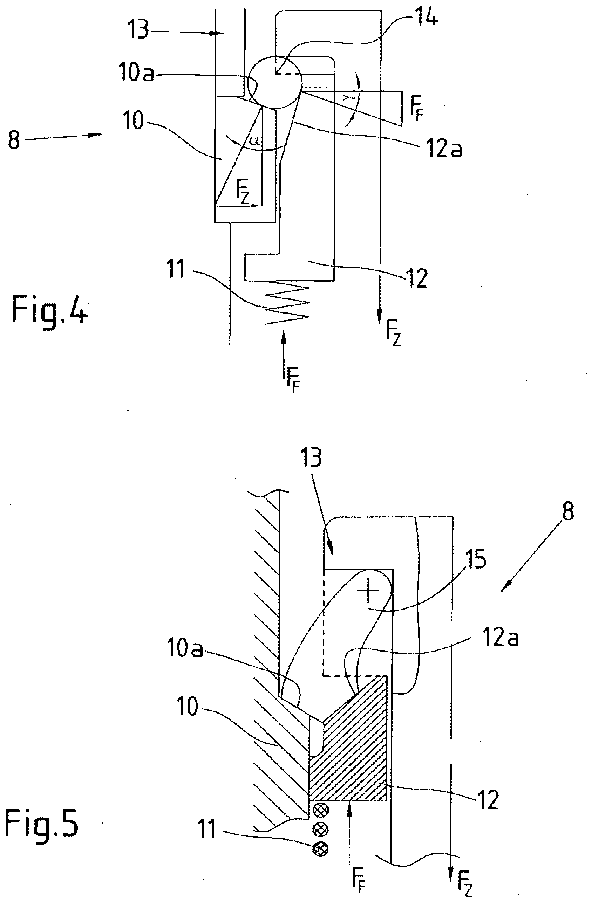

Eine zweite, alternative Ausführungsform zur Ausbildung des Kraftübertragungselements 13 ist in Fig. 5 dargestellt, bei der das Kraftübertragungselement 13 als Schwenkarm 15 ausgebildet ist. Andere Ausführungsformen zur Ausbildung des Kraftübertragungselements 13, wie beispielsweise in der Form eines Kreissegments, sind ebenfalls möglich.

Die Übertragung der Zugkraft F_{Z} des verstellbaren Griffteils 3b der Handhabe 3 über das Kraftübertragungselement 13 auf die beiden Bauteile 10, 12 erfolgt bei der in Fig. 2 und 3 dargestellten Ausführungsform über einen Zuganker 16, der an einem Anlenkpunkt 17 am verstellbaren Griffteil 3b der Handhabe 3 gelagert ist.

Wie aus dem schematischen Kräftediagramm gemäß Fig. 4 ersichtlich, ist die über die Federkraft F_{F} des Federelements 11 einstellbare Grenzlast zum Auslösen der Kraftbegrenzungsvorrichtung 8 eine Winkelfunktion der Kraft des Kraftübertragungselements 13 auf die schräge Anlagefläche 12a des verschiebbaren Bauteils 12, wobei die Kraft des Kraftübertragungselements 13 auf die schräge Anlagefläche 12a des verschiebbaren Bauteils 12 seinerseits eine Winkelfunktion der über das verstellbare Griffteil 3b der Handhabe 3 auf die schräge Anlagefläche 10a des starren Bauteils 10 ausgeübten Zugkraft F_{Z} ist.

Die dargestellte Kraftbegrenzungsvorrichtung 8 arbeitet wie folgt:
Beim Verschwenken des verschwenkbaren Griffteils 3b der Handhabe 3 um die Schwenkachse 5 wird der am Anlenkpunkt 17 angelenkte und im Gehäuse 9 gelagerte Zuganker 16 mitbewegt und infolge der ausgeübten Zugkraft F_{Z} gegen die Federkraft F_{F} des Federelements 11 nach unten gezogen.

Diese Bewegung bewirkt, dass der am oberen Ende des Gehäuses 9 eingehängte und um die Schwenkachse 5 verschwenkbare Kipphebel 7 verschwenkt wird und über eine formschlüssige Einhängung das Zug-/Druckelement 6 zum Schließen des verschwenkbaren Maulteils 4b des Werkzeugs 4 nach proximal zieht.

Kann nun, beispielsweise durch das Ergreifen eines Gegenstandes mit den Maulteilen 4a und 4b des Werkzeugs 4, das verschwenkbare Maulteil 4b nicht weiter geschlossen werden und somit auch das Zug-/Druckelement 6 nicht weiter nach proximal gezogen werden, so steigt aufgrund der Lagerung des Kipphebels 7 am Gehäuse 9 die Zugkraft F_{Z} am Gehäuse.

Nach Überschreiten der über die Federkraft F_{F} des Federelements 11 individuell einstellbaren Grenzlast löst die Kraftbegrenzungsvorrichtung 8 schlagartig aus und die Zugkraft F_{Z} sinkt quasi auf Null ab. In dieser Position kann der verschwenkbare Griffteil 3b der Handhabe 3 bis zu einem Endanschlag 18 weiter verschwenkt werden, jedoch ohne den Kipphebel 5 und somit das Zug-/Druckelement 6 weiter zu betätigen.

Für das schlagartige Auslösen der Kraftbegrenzungsvorrichtung 8 verantwortlich ist das Zusammenspiel der beiden hülsenförmig ausgebildeten Bauteile 10 und 12 mit dem Kraftübertragungselement 13, über das die Zugkraft F_{Z} auf die schrägen Anlageflächen 10a und 12a des starren Bauteils 10 sowie des gegen die Federkraft F_{F} axial verschiebbaren Bauteils 12 übertragen wird.

Die vorgespannte Feder 11 drückt von unten mit der Federkraft F_{F} gegen das verschiebbare Bauteil 12, während das Kraftübertragungselement 13 über die vom verschwenkbaren Griffteil 3b erzeugte Zugkraft F_{Z} von oben auf die schräge Anlagefläche 12a des verschiebbaren Bauteils 12 drückt und dabei radiale Kräfte erzeugt. Das Kraftübertragungselement 13 drückt gleichzeitig von oben auf die schräge Anlagefläche 10a des starr im Gehäuse 9 angeordneten Bauteils 10 und erzeugt dabei ebenfalls radiale Kräfte. Das Kraftübertragungselement 13 kann dem Druck aufgrund der Anordnung im Gehäuse 9 der Kraftbegrenzungsvorrichtung 8 nicht nach radial ausweichen.

Nun wird aber die auf den verschwenkbaren Griffteil 3b aufgebrachte Zugkraft F_{Z} über eine Winkelfunktion in eine Normalkraft und eine Querkraft auf die bzw. entlang der Fläche, auf der das Kraftübertragungselement 13 auf dem Bauteil 10 aufliegt, aufgeteilt, die hieraus resultierende Querkraft wiederum über eine Winkelfunktion in eine Kraft senkrecht zur Zugkraft F_{Z}, die wiederum auf die Fläche des Bauteils 11 wirkt, auf der das Kraftübertragungselement 13 auf dem Bauteil 11 aufliegt. Hier wird die resultierende Kraft auf das Bauteil 11 wiederum über eine Winkelfunktion in eine Normalkraft und eine Querkraft auf die bzw. entlang der Fläche, auf der das Kraftübertragungselement 13 auf dem Bauteil 11 aufliegt, aufgeteilt. Diese Normalkraft auf die Fläche, auf der das Kraftübertragungselement 13 auf dem Bauteil 11 aufliegt, wiederum über eine Winkelfunktion u. a. in eine der Federkraft F_{F} entgegengesetzte Kraft aufgeteilt.

Wird diese der Federkraft F_{F} entgegengesetzte Kraft, quasi die Funktion der aufgebrachten Zugkraft F_{Z}, größer als die Federkraft F_{F}, die das Federelement 11 auf das verschiebbare Bauteil 12 aufbringt, wird das verschiebbare Bauteil 12 gegen die Kraft des Federelements 11 nach unten gedrückt, so dass das Kraftübertragungselement 13 radial zur Seite ausweichen kann und neben dem starren Bauteil 10 zu liegen kommt. Dieses Aufheben der Anlage des Kraftübertragungselements 13 an den beiden Bauteilen 10 und 12 bewirkt ein Herausziehen des Zugankers 16 nach unten aus dem Gehäuse 9 der Kraftbegrenzungsvorrichtung 8, wodurch andererseits das Federelement 11 zusammengedrückt wird.

Das Herausziehen des Zugankers 16 bewirkt eine Verlängerung des Verschwenkweges des verschwenkbaren Griffteils 3b bis zum Endanschlag 18 bei gleichzeitigen Abfall der Zugkraft F_{Z} quasi auf Null.

Die erhaltene Federspannung des Federelements 11 ist ausreichend, um beim Zurückschwenken des verschwenkbaren Griffteils 3b in die Ausgangsposition gemäß Fig. 2 das verschiebbare Bauteil 12 sowie das Kraftübertragungselement 13 reversibel wieder zurück in die Ausgangsposition zu bringen, so dass die Kraftbegrenzungsvorrichtung 8 erneut einsatzbereit ist.

Durch das schlagartige Auslösen der Kraftbegrenzungsvorrichtung 8 ist das Überschreiten der Grenzlast und das Auslösen der Kraftbegrenzungsvorrichtung 8 für den Benutzer nicht nur taktil spürbar, sondern auch akustisch durch deutlich vernehmbares Schlaggeräusch wahrnehmbar.

Bei der in den Abbildungen Fig. 2 und 3 dargestellten Ausführungsform ist die Kraftbegrenzungsvorrichtung 8 zusätzlich mit einer Kappe 19 ausgestattet, die beim Auslösen der Kraftbegrenzungsvorrichtung 8 aus dem verschwenkbaren Griffteil 3b der Handhabe 3 heraustritt, um dem Benutzer das Überschreiten der Grenzlast und das Auslösen der Kraftbegrenzungsvorrichtung 8 auch visuell anzuzeigen.

Ein solchermaßen ausgebildetes medizinisches Instrument 1 zeichnet sich dadurch aus, dass die reversibel arbeitende Kraftbegrenzungsvorrichtung 8 die Zugkraft F_{Z} schlagartig auf Null absinken lässt und dem Benutzer zweifelsfrei anzeigt, dass die Grenzlast überschritten wurde.

**Bezugszeichenliste**

| | | | |
|---|---|---|---|
| 1 | medizinisches Instrument | 12 | verschiebbares Bauteil |
| 2 | Schaft | 12a | Anlagefläche |
| 3 | Handhabe | 13 | Kraftübertragungselement |
| 3a | starres Griffteil | 14 | Kugel |
| 3b | verschwenkbares Griffteil | 15 | Schwenkhebel |
| 3c | Fingeröse | 16 | Zuganker |
| 4 | Werkzeug | 17 | Anlenkpunkt |
| 4a | starres Maulteil | 18 | Endanschlag |
| 4b | verschwenkbares Maulteil | 19 | Kappe |
| 5 | Schwenkachse | | |
| 6 | Zug-/Druckelement | F_{F} | Federkraft |
| 7 | Kipphebel | F_{Z} | Zugkraft |
| 8 | Kraftbegrenzungsvorrichtung | | |
| 9 | Gehäuse | | |
| 10 | starres Bauteil | | |
| 10a | Anlagefläche | | |
| 11 | Federelement | | |

## Patentansprüche

1. Medizinisches Instrument mit einem Schaft (2), an dessen distalem Ende ein mindestens ein bewegbares Maulteil (4b) aufweisendes Werkzeug (4) angeordnet ist und an dessen proximalem Ende eine mit mindestens einem bewegbaren Griffteil (3b) versehene Handhabe (3) angeordnet ist, wobei das bewegbare Maulteil (4b) sowie das bewegbare Griffteil (3b) über ein Zug- / Druckelement (6) miteinander in Wirkverbindung stehen und wobei zwischen dem bewegbaren Griffteil (3b) und dem Zug- / Druckelement (6) mindestens eine Kraftbegrenzungsvorrichtung (8) zum Begrenzen der bei der Kraftübertragung übertragenen Kraft von dem bewegbaren Griffteil (3b) auf das Zug- / Druckelement (6) angeordnet ist, die bei Überschreitung einer Grenzlast auslöst und die miteinander gekoppelten Komponenten bewegbares Griffteil (3b) und Zug- / Druckelement (6) reversibel trennt,
**dadurch gekennzeichnet,**
**dass** die Kraftbegrenzungsvorrichtung (8) ein Gehäuse (9) umfasst, in dem zwei relativ zueinander verschiebbare Bauteile (10, 12) so angeordnet sind, dass ein Bauteil (10) starr im Gehäuse (9) gelagert ist und das andere Bauteil (12) gegen die Kraft eines im Gehäuse (9) angeordneten Federelements (11) axial verschiebbar im Gehäuse (9) angeordnet ist, wobei beide Bauteile (10, 12) über mindestens ein auf beide Bauteile (10, 12) einwirkendes Kraftübertragungselement (13) miteinander gekoppelt sind,
**dass** das mindestens eine Kraftübertragungselement (13) über einen am verstellbaren Griffteil (3b) der Handhabe (3) gelagerten Zuganker (16) mit dem verstellbaren Griffteil (3b) der Handhabe (3) gekoppelt ist,
**dass** das mindestens eine Kraftübertragungselement (13) als Kugel (14) ausgebildet ist,
**dass** Anlageflächen (10a, 12a), an denen das mindestens eine Kraftübertragungselement (13) an den beiden Bauteilen (10, 12) anliegt, als aufeinander zu geneigte Schrägflächen ausgebildet sind,
**dass** die Grenzlast zum Auslösen der Kraftbegrenzungsvorrichtung (8) eine Winkelfunktion der Kraft des Kraftübertragungselements (13) auf die schräge Anlagefläche (12a) des verschiebbaren Bauteils (12) ist, wobei die Kraft des Kraftübertragungselements (13) auf die schräge Anlagefläche (12a) des verschiebbaren Bauteils (12) eine Winkelfunktion der über das verstellbare Griffteil (3b) der Handhabe (3) auf die schräge Anlagefläche (10a) des starren Bauteils (10) ausgeübten Zugkraft (F_{Z}) ist
und
**dass** das Auslösen der Kraftbegrenzungsvorrichtung (8) taktil und / oder akustisch und / oder optisch wahrnehmbar ist.

2. Medizinisches Instrument nach Anspruch 1, **dadurch gekennzeichnet, dass** über das mindestens eine Kraftübertragungselement (13) die Zugkraft (F_{Z}) des verstellbaren Griffteils (3b) der Handhabe (3) auf die beiden Bauteile (10, 12) übertragbar ist.

3. Medizinisches Instrument nach einem der Ansprüche 1 bis 2, **dadurch gekennzeichnet, dass** die Kraftbegrenzungsvorrichtung (8) im bewegbaren Griffteil (3b) der Handhabe (3) im Bereich des Kraftübertragungsmechanismus auf das Zug- / Druckelement (6) angeordnet ist.

4. Medizinisches Instrument nach einem der Ansprüche 1 bis 3, **dadurch gekennzeichnet, dass** die das Auslösen der Kraftbegrenzungsvorrichtung (8) bewirkende Grenzlast über das auf das verschiebbare Bauteil (12) einwirkende Federelement (11) einstellbar ist.

5. Medizinisches Instrument nach einem der Ansprüche 1 bis 4, **dadurch gekennzeichnet, dass** die beiden über das mindestens eine Kraftübertragungselement (13) miteinander gekoppelten Bauteile (10, 12) hülsenförmig ausgebildet sind.

6. Medizinisches Instrument nach einem der Ansprüche 1 bis 5, **dadurch gekennzeichnet, dass** die Kraftbegrenzungsvorrichtung (8) die über das verstellbare Griffteil (3b) der Handhabe (3) aufgebrachte Zugkraft (F_{Z}) im Auslösefall im Wesentlichen auf Null herabsetzt.

7. Medizinisches Instrument nach einem der Ansprüche 1 bis 6, **dadurch gekennzeichnet, dass** die Kraftbegrenzungsvorrichtung (8) im Bereich des Zug- / Druckelements (6) angeordnet ist.

## Claims

1. Medical instrument with a shaft (2), at the distal end of which is arranged a tool (4) having at least one movable jaw part (4b), and at the proximal end of which is arranged a handle (3) provided with at least one movable grip part (3b), the movable jaw part (4b) and the movable grip part (3b) being operatively connected to each other via a pull / push element (6), and at least one force-limiting device (8) being arranged between the movable grip part (3b) and the pull / push element (6) in order to limit the force transmitted in the force transmission from the movable grip part (3b) to the pull / push element (6), said force-limiting device (8) being triggered when a maximum load is exceeded and reversibly separating the components coupled to each other, namely the movable grip part (3b) and pull / push element (6), **characterized in that**
the force-limiting device (8) comprises a housing (9) in which two components (10, 12), displaceable relative to each other, are arranged such that one component (10) is mounted rigidly in the housing (9) and the other component (12) is arranged to be axially displaceable in the housing (9) counter to the force of a spring element (11) arranged in the housing (9), the two components (10, 12) being coupled to each other via at least one force transmission element (13) that acts on both components (10, 12),
the at least one force transmission element (13) is coupled to the adjustable grip part (3b) of the handle (3) via a tie rod (16) mounted on the adjustable grip part (3b) of the handle (3),
the at least one force transmission element (13) is designed as a ball (14),
contact surfaces (10a, 12a), on which the at least one force transmission element (13) bears on the two components (10, 12), are configured as oblique surfaces slanting towards each other,
the maximum load for triggering the force-limiting device (8) is an angle function of the force of the force transmission element (13) on the oblique contact surface (12a) of the displaceable component (12), said force of the force transmission element (13) on the oblique contact surface (12a) of the displaceable component (12) being an angle function of the tensile force (F_{Z}) exerted on the oblique contact surface (10a) of the rigid component (10) via the adjustable grip part (3b) of the handle (3), and
the triggering of the force-limiting device (8) is discernible by tactile and/or acoustic and/or optical means.

2. Medical instrument according to Claim 1, **characterized in that** the tensile force (F_{Z}) of the adjustable grip part (3b) of the handle (3) can be transmitted to the two components (10, 12) via the at least one force transmission element (13).

3. Medical instrument according to one of Claims 1 to 2, **characterized in that** the force-limiting device (8) is arranged in the movable grip part (3b) of the handle (3) in the area of the mechanism for force transmission to the pull / push element (6).

4. Medical instrument according to one of Claims 1 to 3, **characterized in that** the maximum load that triggers the force-limiting device (8) is adjustable via the spring element (11) acting on the displaceable component (12).

5. Medical instrument according to one of Claims 1 to 4, **characterized in that** the two components (10, 12) coupled to each other via the at least one force transmission element (13) have a sleeve-shaped configuration.

6. Medical instrument according to one of Claims 1 to 5, **characterized in that**, when triggered, the force-limiting device (8) reduces the tensile force (F_{Z}) applied via the adjustable grip part (3b) of the handle (3) substantially to zero.

7. Medical instrument according to one of Claims 1 to 6, **characterized in that** the force-limiting device (8) is arranged in the area of the pull / push element (6).

## Revendications

1. Instrument médical avec une tige (2), à l'extrémité distale de laquelle est disposé un outil (4) présentant au moins une partie de mâchoire (4b) pouvant être déplacée et à l'extrémité proximale de laquelle est disposée une poignée (3) pourvue d'au moins une partie de manche (3b) pouvant être déplacée, où la partie de mâchoire (4b) pouvant être déplacée ainsi que la partie de manche (3b) pouvant être déplacée sont en liaison active l'une avec l'autre par l'intermédiaire d'un élément de traction et de pression (6) et où au moins un dispositif de limitation de la force (8), destiné à la limitation de la force exercée lors de la transmission de la force de la partie de manche (3b) pouvant être déplacée sur l'élément de traction et de pression (6), est disposé entre la partie de manche (3b) pouvant être déplacée et l'élément de traction et de pression (6), lequel dispositif de limitation de la force se déclenche quand une charge limite est dépassée et sépare de manière réversible ces composants couplés l'un à l'autre, à savoir la partie de manche (3b) pouvant être déplacée et l'élément de traction et de pression (6),
**caractérisé en ce que**
le dispositif de limitation de la force (8) comprend un boîtier (9), dans lequel deux éléments structurels (10, 12) sont disposés de manière mobile l'un par rapport à l'autre, de telle sorte qu'un élément structurel (10) est mis en place de manière fixe dans le boîtier (9) et que l'autre élément structurel (12) est disposé dans le boîtier (9), de manière à pouvoir être mobile dans la direction axiale, contre la force d'un élément à ressort (11) disposé dans le boîtier (9), où les deux éléments structurels (10, 12) sont couplés l'un avec l'autre par l'intermédiaire d'au moins un élément de transmission de la force (13) qui agit sur les deux éléments structurels (10, 12) ;
**caractérisé en ce que** l'au moins un élément de transmission de la force (13) est couplé avec la partie de manche (3b) ajustable de la poignée (3) par l'intermédiaire d'un tirant d'ancrage (16) mis en place au niveau de la partie de manche (3b) ajustable de la poignée (3) ;
**caractérisé en ce que** l'au moins un élément de transmission de la force (13) est conçu sous la forme d'une boule (14) ;
**caractérisé en ce que** des surfaces d'appui (10a, 12a) sont conçues sous la forme de surfaces inclinées, lesquelles sont inclinées l'une vers l'autre, surfaces d'appui au niveau desquelles l'au moins un élément de transmission de la force (13) repose au niveau des deux éléments structurels (10, 12) ;
**caractérisé en ce que** la charge limite destinée au déclenchement du dispositif de limitation de la force (8) est une fonction angulaire de la force exercée par l'élément de transmission de la force (13) sur la surface d'appui (12a) inclinée de l'élément structurel (12) mobile, où la force exercée par l'élément de transmission de la force (13) sur la surface d'appui (12a) inclinée de l'élément structurel (12) mobile est une fonction angulaire de la force de traction (F_{Z}) exercée sur la surface d'appui (10a) inclinée de l'élément structurel (10) immobile par l'intermédiaire de la partie de manche (3b) ajustable de la poignée (3) ; et
**caractérisé en ce que** le déclenchement du dispositif de limitation de la force (8) est perceptible de manière tactile et / ou acoustique et / ou optique.

2. Instrument médical selon la revendication 1, **caractérisé en ce que** la force de traction (F_{Z}) de la partie de manche (3b) ajustable de la poignée (3) peut être transmise sur les deux éléments structurels (10, 12) par l'intermédiaire de l'au moins un élément de transmission de la force (13).

3. Instrument médical selon l'une des revendications 1 à 2, **caractérisé en ce que** le dispositif de limitation de la force (8) est disposé sur l'élément de traction et de pression (6) dans la zone du mécanisme de transmission de la force, dans la partie de manche (3b) pouvant être déplacée de la poignée (3).

4. Instrument médical selon l'une des revendications 1 à 3, **caractérisé en ce que** la charge limite provoquant le déclenchement du dispositif de limitation de la force (8) peut être réglée par l'intermédiaire de l'élément à ressort (11) agissant sur l'élément structurel (12) mobile.

5. Instrument médical selon l'une des revendications 1 à 4, **caractérisé en ce que** les deux éléments structurels (10, 12), qui sont couplés l'un à l'autre par l'intermédiaire de l'au moins un élément de transmission de la force (13), sont conçus en forme de manchon.

6. Instrument médical selon l'une des revendications 1 à 5, **caractérisé en ce que** le dispositif de limitation de la force (8), dans le cas d'un déclenchement, ramène pour l'essentiel à zéro la force de traction (F_{Z}) appliquée par l'intermédiaire de la partie de manche (3b) ajustable de la poignée (3).

7. Instrument médical selon l'une des revendications 1 à 6, **caractérisé en ce que** le dispositif de limitation de la force (8) est disposé dans la zone de l'élément de traction et de pression (6).
